# FASCICULE DE BREVET EUROPEEN

(11) **EP 1 372 541 B1**
(45) Date de publication et mention de la délivrance du brevet: **30.03.2011**
(21) Numéro de dépôt: 02730327.0
(22) Date de dépôt: 03.04.2002
(51) Int. Cl.: A61F 2/44

(54) **SYSTEME STABILISE DE FUSION INTERSOMATIQUE POUR VERTEBRES**
STABILISIERUNGSSYSTEM FÜR DEN ZWISCHENWIRBELBEREICH
STABILISED INTERBODY FUSION SYSTEM FOR VERTEBRAE

(30) Priorité: 03.04.2001 FR 0104489
(43) Date de publication de la demande: 02.01.2004
(73) Titulaire: SCIENT'X, 78284 Guyancourt (FR)
(72) Inventeur: FIERE, Vincent, F-69006 Lyon (FR); FAYADA, Paul, F-62600 Berck (FR); TAZIAUX, Jean-Paul, F-62150 Rebreuve-Ranchicourt (FR)
(74) Mandataire: Thibault, Jean-Marc
(86) Numéro de dépôt international: PCT/FR2002/001157
(87) Numéro de publication internationale: WO 2002/080819

(56) Documents cités:
- EP-A- 0 974 319
- WO-A-00/24343
- WO-A-98/48718
- FR-A- 2 727 005

## Description

La présente invention concerne le domaine technique des implants intersomatiques, destinés à être insérés, après excision discale, dans l'espace intervertébral défini entre deux vertèbres adjacentes, en vue de restituer l'espace intervertébral et d'assurer une fusion osseuse entre lesdites vertèbres adjacentes.

L'objet de l'invention vise, plus précisément, le domaine des implants intersomatiques, du type lombaire, destinés à rétablir la hauteur et l'angle de lordose du segment vertébral défini par deux vertèbres lombaires voisines à solidariser.

L'objet de invention vise, plus précisément, un implant intersomatique, dit sécurisé, c'est-à-dire équipé de moyens assurant l'ancrage de l'implant dans son site d'insertion, en vue d'éviter sa mobilisation.

La demande de brevet EP 0 974 319 décrit un implant de reconstruction vertébrale destiné à remplacer des vertèbres ou un disque intervertébral. Cet implant comporte un corps de reconstruction relié à une plaque de stabilisation à l'aide d'un vis d'assemblage.

Dans l'état de la technique, il est connu plusieurs systèmes stabilisés de fusion intersomatique pour vertèbres. Par exemple, il est connu par le document WO 00 24343 un système de fusion intersomatique comportant un implant intervertébral présentant une partie bombée prolongée par un axe fileté de réception d'une plaque de stabilisation apte à venir en appui sur la partie bombée de la cage afin d'autoriser une angulation relative entre l'implant et la plaque.

Il est connu aussi par la demande de brevet européen 0 891 169 un système stabilisé comportant un implant intersomatique, destiné à être inséré dans l'espace intervertébral défini entre deux vertèbres lombaires adjacentes. Selon une forme préférée de réalisation, cet implant se présente sous la forme d'une cage comportant deux parois sagittales reliées entre elles par une paroi transversale antérieure et par une paroi transversale postérieure. Les parois délimitent entre elles un volume s'ouvrant de part et d'autre des faces transversales de l'implant. Le volume ouvert de la cage est destiné à recevoir un produit de comblement osseux, appelé greffon osseux, destiné à venir en contact avec les plateaux vertébraux permettant de favoriser la fusion osseuse entre les deux vertèbres.

Un tel système stabilisé comporte, également, une plaque de stabilisation s'étendant à partir de la paroi antérieure de l'implant et de part et d'autre des faces transversales. Cette plaque de stabilisation est munie, à chacune de ses extrémités, de trous de passage pour des vis destinées à être ancrées dans les vertèbres à solidariser.

La mise en place d'une plaque de stabilisation permet d'éviter, de façon sûre, une migration de l'implant. Toutefois, la déposante a mis en évidence une difficulté à maintenir l'implant dans sa position idéale à l'intérieur de l'espace intervertébral, tout en permettant la fixation de la plaque de stabilisation sur les vertèbres. En effet, il doit être considéré que d'un patient à l'autre, d'une part, les bords antérieurs des plateaux des corps des vertèbres présentent des profils variables et, d'autre part, les implants se situent à des distances variables par rapport aux bords antérieurs des plateaux des corps des vertèbres, ce qui conduit à un déplacement de l'implant lors de l'opération de fixation de la plaque de stabilisation sur les vertèbres.

La déposante a exprimé le besoin de disposer d'un système stabilisé de fusion intersomatique, conçu pour permettre, d'une part, de placer l'implant dans l'espace intervertébral dans une position adaptée pour restaurer une courbure et une lordose rachidienne physiologique et, d'autre part, de maintenir l'implant dans cette position idéale de correction, lors de l'ancrage de la plaque de stabilisation sur les vertèbres, quelle que soit la forme des bords antérieurs des plateaux des corps des vertèbres et la position de l'implant par rapport à ces bords antérieurs des plateaux.

L'objet de l'invention vise donc à satisfaire ce besoin en proposant un système stabilisé de fusion intersomatique adapté pour rétablir la hauteur et l'angle de lordose du segment vertébral par un implant, dont la fonction est sécurisée par une plaque de stabilisation fixée sur les vertèbres.

Pour atteindre cet objectif, le système stabilisé de fusion intersomatique pour vertèbres conforme à l'invention est du type défini dans la revendication 1.

Diverses autres caractéristiques ressortent de la description faite ci-dessous en référence aux dessins annexés qui montrent, à titre d'exemples non limitatifs, des formes de réalisation de l'objet de l'invention.

La fig. 1 est une vue en perspective éclatée montrant les différents éléments constitutifs du système stabilisé selon l'invention.

La fig. 2 est une vue en coupe-élévation d'un système stabilisé en position montée.

La fig. 3 est une vue en élévation montrant un système stabilisé monté sur deux vertèbres.

La fig. 4 est une vue en coupe-élévation montrant une autre variante de réalisation du système stabilisé conforme à l'invention.

Les fig. 5 et 6 sont des vues en perspective montrant des détails caractéristiques d'un instrument de préhension d'un implant conforme à l'invention.

La fig. 7 est une vue en coupe-élévation illustrant une autre variante de réalisation d'un système stabilisé conforme à l'invention.

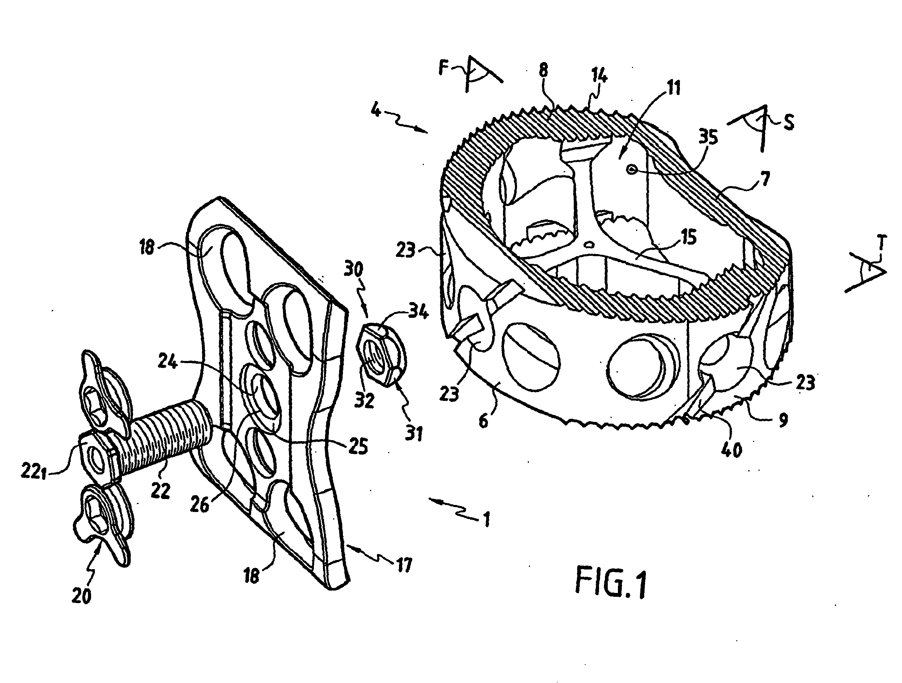

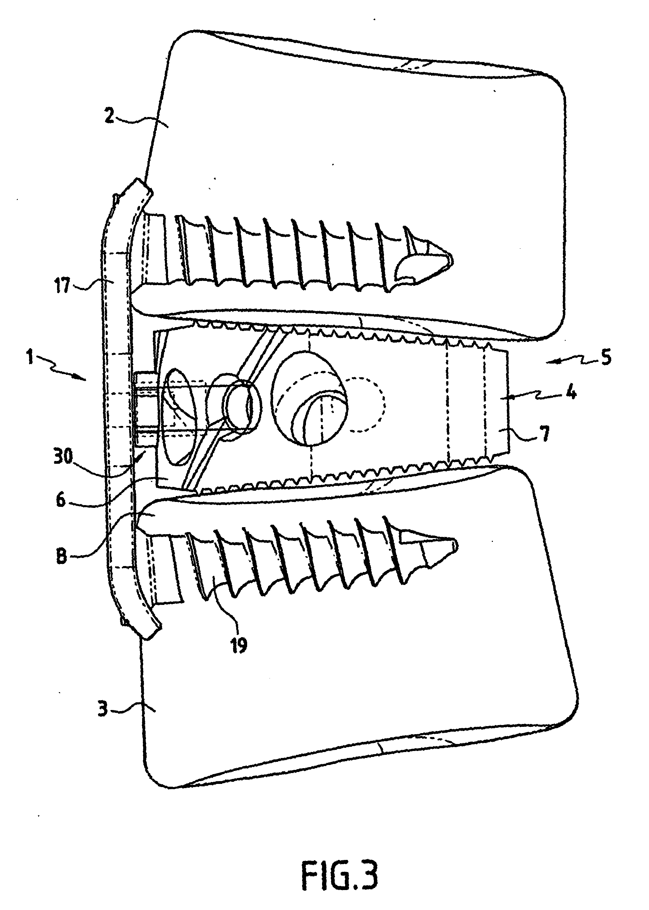

Tel que cela ressort plus précisément des fig. 1 à 3, l'objet de l'invention concerne un système stabilisé 1 de fusion intersomatique pour deux vertèbres voisines ou adjacentes 2, 3. Le système stabilisé 1 comporte un implant intersomatique 4, destiné à être inséré dans l'espace intervertébral 5 délimité par les deux vertèbres 2, 3. Dans un exemple préféré de réalisation, tel qu'illustré sur les dessins, l'implant 4 est réalisé par une cage présentant une paroi transversale 6, dite antérieure, et une paroi transversale 7, dite postérieure, reliées entre elles par des parois de raccordement 8, 9. Les parois 6 à 9 de la cage s'élèvent perpendiculairement par rapport à un plan transversal T qui est perpendiculaire à un plan sagittal S passant par les parois antérieure 6 et postérieure 7. Dans le plan transversal T, la paroi antérieure 6 présente une courbe convexe et se trouve prolongée de part et d'autre par les parois de raccordement 8, 9, de courbures également convexes, reliées entre elles par la paroi postérieure 7 de courbure concave pour laisser dégager le canal vertébral. La cage 1 présente ainsi une forme générale réniforme.

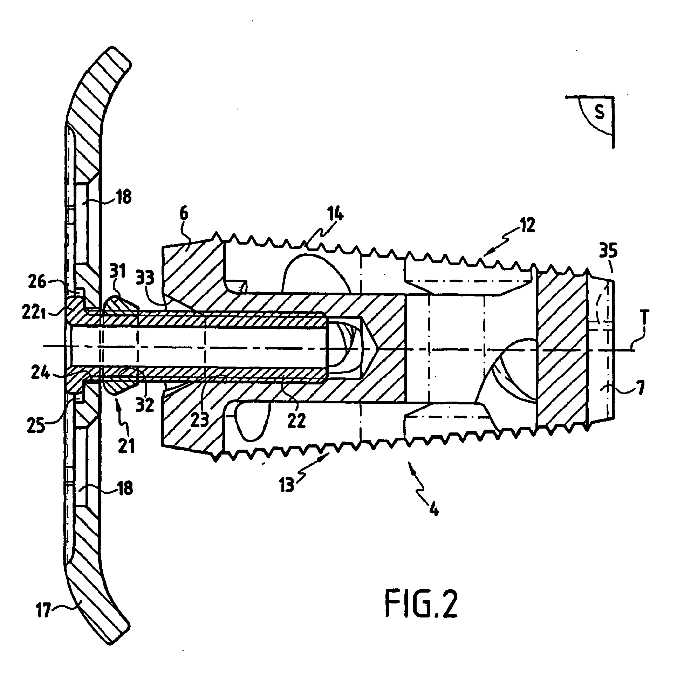

Tel que cela ressort plus précisément de la fig. 2, les parois de raccordement 8, 9 dans le plan sagittal S convergent entre elles en direction de la paroi postérieure 7, de sorte que la cage 1 possède une section tronconique dans le plan sagittal S, permettant de définir un angle de rétablissement de lordose.

La cage 1 présente, intérieurement, un volume 11 délimité par les faces verticales intérieures des parois 6 à 9 et est destiné à être rempli par un produit de comblement osseux, appelé greffon osseux, adapté à la fusion intersomatique. Ce volume 11 s'ouvre selon une première face transversale 12, dite supérieure dans l'exemple illustré, et une deuxième face transversale 13, dite inférieure. Ces faces transversales 12, 13 sont délimitées par les rebords des parois 6 à 9 aménagées, de préférence, pour comporter des crans 14 pour permettre un accrochage de la cage sur les vertèbres sus et sous-jacentes. Les crans 14 s'étendent parallèlement les uns aux autres et par rapport à un plan frontal F perpendiculaire au plan sagittal S et au plan transversal T.

De préférence, le volume interne 11 de la cage comporte une ou plusieurs parois de liaison 15 avec les autres parois. Dans l'exemple illustré, la paroi de liaison 15 présente une forme générale en "Y" et s'étend entre les parois de raccordement 8 et 9 et la paroi antérieure 6.

Le système stabilisé de fusion intersomatique 1 conforme à l'invention comporte, également, une plaque 17 de stabilisation pour l'implant 4. Cette plaque de stabilisation 17 est munie, à chacune de ses extrémités, d'au moins un et dans l'exemple illustré de deux trous 18 de passage pour des vis 19 destinées à être ancrées dans les vertèbres 2, 3 à solidariser entre elles.

Selon une caractéristique préférée de réalisation, la plaque de stabilisation 17 est équipée de moyens d'anti-expulsion 20 pour les vis d'ancrage 19. Ces moyens d'anti-expulsion 20 sont constitués par des volets mobiles, montés sur la plaque de stabilisation pour être guidés en rotation, en vue d'occuper une première position dans laquelle la section de passage des trous 18 est laissée libre et une seconde position dans laquelle cette section de passage est obturée, de manière à constituer une butée pour les têtes des vis 19.

La plaque de stabilisation 17 et l'implant 4 sont équipés de moyens d'assemblage 21 permettant d'assurer leur assemblage entre eux. La plaque de stabilisation 17 est adaptée de manière, qu'après assemblage avec l'implant 4, elle s'étend de part et d'autre des faces transversales 12, 13, en vue de permettre l'ancrage des vis 19 dans les vertèbres 2, 3.

Selon une caractéristique de réalisation, les moyens d'assemblage 21 sont réalisés par une vis d'assemblage 22 destinée à coopérer avec au moins un trou taraudé 23 aménagé dans l'implant 4. Par exemple, un trou taraudé 23 est aménagé pour traverser la paroi antérieure 6 de part et d'autre, selon un axe perpendiculaire à la paroi antérieure 6 et centré sur le plan sagittal S de symétrie de l'implant 4. De préférence, l'implant 4 comporte une série de trous taraudés 23, de manière à autoriser le montage de la plaque de stabilisation 17 selon diverses positions par rapport à l'implant 4. Ainsi, un trou taraudé 23 est aménagé dans une paroi de raccordement, par exemple 9, selon un axe perpendiculaire à l'axe du trou taraudé 23 aménagé dans la paroi antérieure 6. Par les positions définies par ces deux trous taraudés 23, la plaque de stabilisation 17 est apte à occuper deux positions perpendiculaires entre elles. Un autre trou taraudé 23 peut aussi être aménagé dans l'autre paroi de raccordement, à savoir 8, selon un axe faisant un angle de 45° avec l'axe du trou taraudé 23 aménagé dans la paroi antérieure 6.

La vis d'assemblage 22 traverse la plaque de stabilisation 17 par un trou de passage 24, bordé par un épaulement 25 de butée réalisé par une cuvette 26 aménagée pour recevoir la tête 221 de la vis d'assemblage 22.

Selon une caractéristique de l'invention, le dispositif stabilisé 1 comporte des moyens d'écartement 30, interposés entre la plaque de stabilisation 17 et l'implant 4, pour permettre de positionner, à une distance déterminée, la plaque de stabilisation 17 par rapport à l'implant 4. Ces moyens d'écartement 30 permettent de fixer la plaque de stabilisation 17 sur les vertèbres en laissant l'implant 4 dans sa position, tout en tenant compte de la forme des bords antérieurs B des plateaux des corps des vertèbres 2, 3 ou du positionnement en retrait de l'implant 4 par rapport à ces bords antérieurs des vertèbres.

Dans l'exemple illustré sur les fig. 1 à 3, les moyens d'écartement 30 sont constitués par une douille d'entretoisement 31 pourvue d'un taraudage 32 pour coopérer avec le filetage de la vis d'assemblage 22. La douille d'entretoisement 31 est ainsi réalisée sous la forme d'un écrou et comporte, extérieurement, une forme prismatique adaptée pour coopérer avec une partie de réception 33 de l'implant 4 dans laquelle débouche le trou taraudé 23. La coopération de la douille d'entretoisement 31 avec la partie de réception 33 permet de bloquer en rotation la douille 31 lors du vissage de la vis d'assemblage 22.

De préférence, la partie de réception 33 est réalisée par un cône d'entrée coopérant avec une partie complémentaire 34 présentée par la douille 31. La coopération de douille 31 avec le cône d'entrée 33 permet d'assurer le réglage de la distance entre la plaque de stabilisation 17 et l'implant 4.

La mise en oeuvre du système stabilisé 1 selon l'invention découle directement de la description qui précède.

Préalablement à l'insertion de l'implant 4, il est procédé à l'excision discale suivie de la préparation des plateaux vertébraux. L'implant 4 est implanté dans l'espace intervertébral 5 défini entre les deux vertèbres voisines 2 et 3 à solidariser entre elles. L'implant 4 est destiné à rétablir la hauteur et l'angle de lordose du segment vertébral défini par les deux vertèbres voisines 2, 3. La plaque de stabilisation 17, équipée de la vis d'assemblage 22 pourvue de sa douille d'entretoisement 31, est destinée à être montée sur l'implant 4.

La vis 22 est alors vissé dans un trou taraudé 23 de l'implant. Le vissage de la vis 22 est alors assuré jusqu'à obtenir son blocage par la mise en butée de la douille d'entretoisement 31, entre la plaque 17 et l'implant 4. Les vis 19 sont alors ancrées dans les vertèbres 2, 3.

Bien entendu, la douille d'entretoisement 31 présente une longueur adaptée pour permettre de laisser l'implant 4 dans son site d'implantation, tout en permettant à la plaque de stabilisation 17 de venir en appui sur les vertèbres. La plaque de stabilisation 17 et l'implant 4 se trouvent écartés d'une mesure fixe déterminée par la douille d'entretoisement 31. De manière préférée, une gamme de douilles d'entretoisement 31 est disponible. Chacune de ces douilles d'entretoisement 31 présente une longueur déterminée et différente entre elles, de manière à permettre de régler la distance entre l'implant 4 et la plaque de stabilisation 17.

Il est à noter que l'implant 4 est muni de préférence d'au moins un élément radio-opaque 35 (fig. 1 et 2) adapté pour permettre de détecter sur une radiographie, la position de l'implant 4 dans l'espace intervertébral 5 selon le plan sagittal S. Dans l'exemple illustré, l'élément radio-opaque 35 est monté dans la paroi postérieure 7 en s'étendant dans le plan transversal, et en débouchant sur la face externe et la face interne de la paroi postérieure 7.

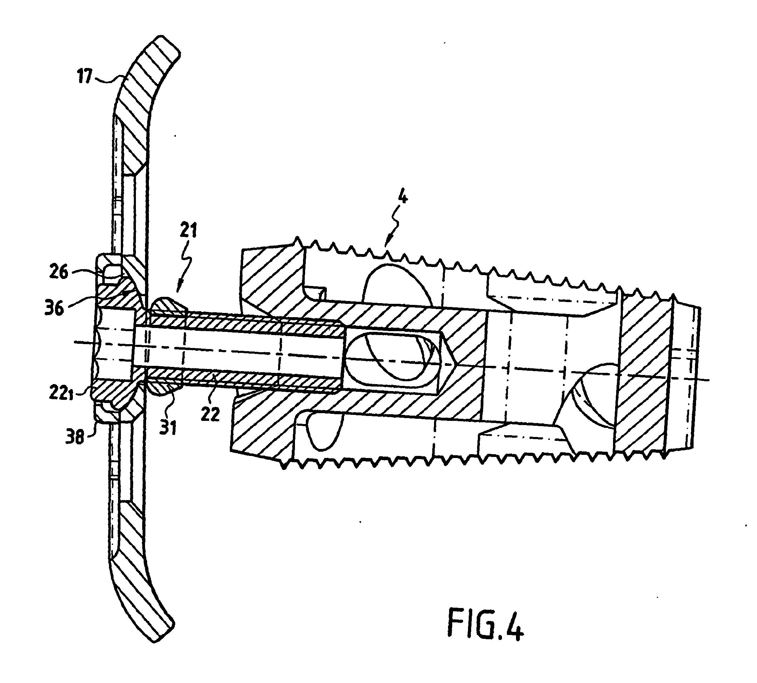

Selon une caractéristique de réalisation illustrée à la fig. 4, les moyens d'assemblage 21 sont pourvus de moyens de support 36 de la plaque de stabilisation 17 autorisant une orientation angulaire de la plaque de stabilisation 17 par rapport à l'implant 4. Ces moyens de support 36 sont réalisés, par exemple, par une articulation du type à rotule constituée entre la tête 221 de la vis d'assemblage 22 et la cuvette 26 de la plaque 17. Dans cette variante de réalisation illustrée, la vis 22 est assemblée à la plaque de stabilisation 17 à l'aide d'une bague 38 fixée sur la plaque 17 pour emprisonner la tête 221 de la vis 22 en laissant à la vis 22 une liberté de rotation sur elle-même.

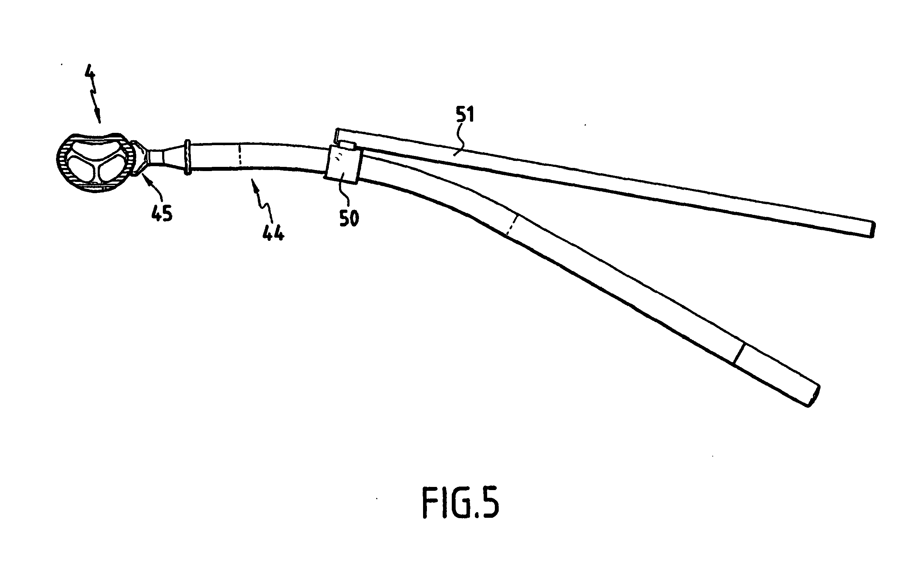

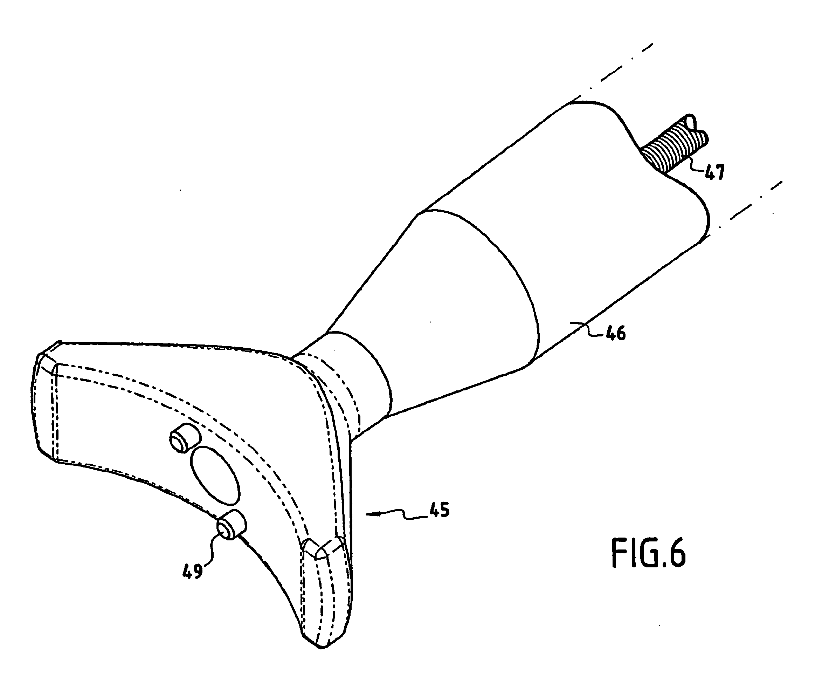

Selon une variante préférée de réalisation, chaque trou taraudé 23 de l'implant est bordé par une rainure d'assemblage 40, inclinée par rapport au plan sagittal et destinée à coopérer avec un instrument de préhension ou de manipulation 44 de l'implant 4. Tel que cela ressort des fig. 5 et 6, l'instrument de manipulation 44 comporte une tête d'assemblage 45, prolongée par un tube de manoeuvre 46 dans lequel est montée une tige filetée flexible 47 traversant la tête 45, en vue de coopérer avec un trou taraudé 23 de l'implant. La tête d'assemblage 45 est munie de deux tétons 49, destinés à venir s'emboîter dans la rainure 40, de part et d'autre du trou taraudé 23. L'instrument 44 permet ainsi d'assurer une préhension et un blocage complet de l'implant 4 par rapport à la tête d'assemblage 45.

Selon une variante préférée de réalisation, le tube de manoeuvre 46 présente une courbure et se trouve équipé d'un coulisseau 50 portant une barre 51 dont l'extrémité libre est adaptée pour recevoir des efforts pour la mise en place de l'implant et le respect de la voie d'abord.

Dans la description qui précède, le système stabilisé de fusion intersomatique 1 comporte un implant 4 réalisé sous la forme d'une cage de forme générale parallélépipèdique. Bien entendu, l'objet de l'invention peut être mis en oeuvre pour un implant intersomatique réalisé sous la forme d'une cage cylindrique ou semi-cylindrique.

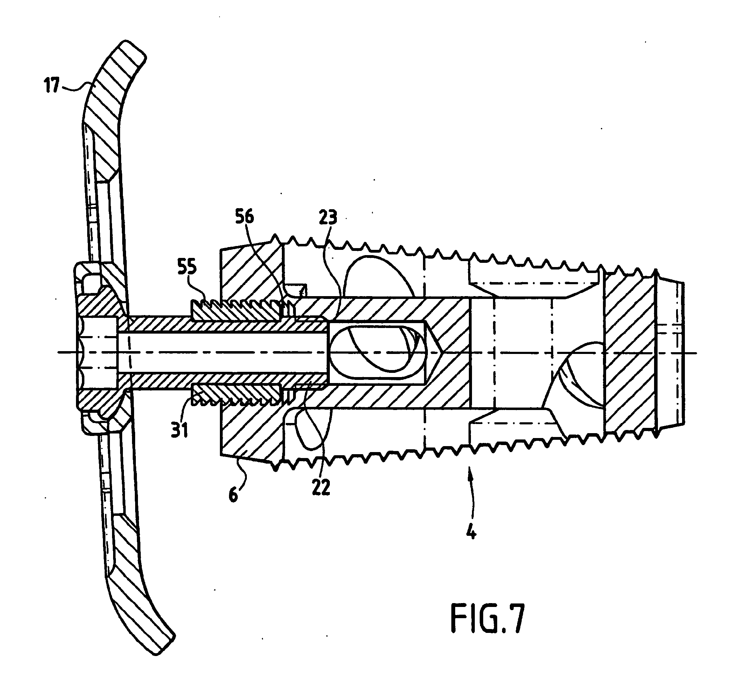

Dans la description qui précède, la douille d'entretoisement 31 est mise en place lors du montage de la plaque de stabilisation 17 sur l'implant 4, en étant portée par la vis d'assemblage 22. Bien entendu, il peut être prévu que les moyens d'écartement 30 se trouvent fixés sur la plaque de stabilisation 17. Dans le même sens, la fig. 7 illustre une autre variante de réalisation de l'invention, dans laquelle la douille d'entretoisement 31 est fixée sur l'implant 4 en per-opératoire. Cette douille d'entretoisement 31 comporte des moyens de montage 55 sur l'implant, réalisés par exemple, par des crans coopérant avec un crantage complémentaire 56 aménagé dans la paroi antérieure 6, et dans lequel débouche le trou taraudé 23. Cette douille d'entretoisement 31 peut donc être montée sur l'implant 4 avec une possibilité de réglage de son degré d'enfoncement, permettant ainsi de régler la distance entre l'implant 4 et la plaque de stabilisation 17. De préférence, cette douille d'entretoisement 31 est fendu sur sa longueur, de manière à obtenir son blocage en position lors du montage de la vis d'assemblage 22, qui traverse cette douille 31 pour être vissée dans le trou taraudé 23.

L'invention n'est pas limitée aux exemples décrits et représentés car diverses modifications peuvent y être apportées sans sortir de son cadre, limité par les revendications suivantes.

## Revendications

1. Système stabilisé de fusion intersomatique pour vertèbres, du type comportant :
un implant intersomatique (4) destiné à être inséré dans l'espace intervertébral (5) défini entre deux vertèbres (2, 3) voisines à solidariser entre elles, en vue du rétablissement de la hauteur et de l'angle de lordose du segment vertébral défini par les deux vertèbres voisines,
une plaque de stabilisation (17) munie, à chacune de ses extrémités, d'au moins un trou de passage (18) pour une vis d'ancrage (19), la plaque (17) et l'implant (4) étant pourvus de moyens (21) d'assemblage entre eux, de manière qu'après assemblage, la plaque de stabilisation (17) s'étende de part et d'autre de l'implant pour permettre l'ancrage, par les vis, de la plaque de stabilisation sur les vertèbres voisines, et des moyens d'écartement (30) interposés entre la plaque de stabilisation (17) et l'implant (4), pour permettre de positionner, à une distance déterminée, la Plaque de stabilisation par rapport à l'implant, **caractérisé en ce que** les moyens d'écartement sont constitués par au moins une douille d'entretoisement (31) et **en ce que** l'implant présente une partie de réception (33,56) pour une partie complémentaire (34, 5) présentée par la douille d'entretoisement (31) mise en butée entre la plaque de stabilisation (17) et l'implant (4) pour définir par sa longueur, la distance entre la plaque de stabilisation (17) et l'implant (4).

2. Système selon la revendication 1, **caractérisé en ce que** la partie de réception (33) est réalisée par un cône d'entrée.

3. Système selon la revendication 1 ou 2, **caractérisé en ce que** la douille d'entretoisement (31) est un écrou présentant une forme prismatique (34) coopérant avec la partie de réception (33) de l'implant (4).

4. Système selon l'une des revendications 1 à 3, **caractérisé en ce que** les moyens d'assemblage (21) sont pourvus de moyens de support de la plaque autorisant une orientation angulaire de la plaque (17) par rapport à l'implant (4).

5. Système selon la revendication 1, **caractérisé en ce que** la douille d'entretoisement (31) est fixée sur la plaque de stabilisation (17).

6. Système selon la revendication 1, **caractérisé en ce que** la douille d'entretoisement (31) comporte des moyens de montage sur l'implant, assurant un réglage de la distance entre la plaque de stabilisation (17) et l'implant (4).

7. Système selon la revendication 6, **caractérisé en ce que** la douille d'entretoisement (31) est fixée sur l'implant (4) et comporte des crans (55) coopérant avec un crantage complémentaire (56) aménagé sur l'implant (4).

8. Système selon la revendication 5, **caractérisé en ce que** la douille d'entretoisement (31) est emmanchée sur une vis d'assemblage (22) faisant partie des moyens d'assemblage (21) et destinée à traverser la plaque par un trou de passage (24) bordé par un épaulement de butée (25) pour la tête (221) de la vis d'assemblage (22) et à coopérer avec au moins un trou taraudé (23) aménagé dans l'implant.

9. Système selon la revendication 8, **caractérisé en ce que** le trou taraudé (23) est aménagé dans la paroi antérieure (6) de l'implant, présentant, dans le plan transversal, une courbure convexe et prolongée par des parois de raccordement (8, 9) de courbure convexe, à une paroi postérieure (7) possédant une courbure concave pour laisser dégagé le canal vertébral.

10. Système selon la revendication 9, **caractérisé en ce que** chaque paroi de raccordement (8, 9) est aménagée pour comporter un trou taraudé (2, 3) bordé, comme le trou taraudé (23) aménagé dans la paroi antérieure (6), par une rainure d'assemblage (40) destinée à coopérer avec deux tétons d'assemblage (49) équipant la tête (45) d'un instrument de préhension (44) de l'implant (4) comportant, également, une tige filetée (47) destinée à coopérer avec un trou fileté taraudé (23) de l'implant.

11. Système selon la revendication 9, **caractérisé en ce que** la paroi postérieure (7) est munie d'un élément radio-opaque (35) débouchant sur la face externe de ladite paroi, pour permettre de détecter la position de l'implant dans l'espace intervertébral (5) selon le plan sagittal (S).

12. Système selon la revendication 1, **caractérisé en ce que** la plaque de stabilisation (17) est équipée de moyens d'anti-expulsion (20) des vis d'ancrage (19), montés sur la plaque (17) par l'intermédiaire de moyens de guidage en déplacement et adaptés pour, dans une première position, laisser libre la section de passage des trous (18) de réception des vis d'ancrage (19) et, dans une seconde position, obturer au moins partiellement les trous (18), de manière à constituer une butée pour les têtes des vis.

## Claims

1. A stabilised interbody fusion system for vertebrae, of the type comprising:
an interbody implant (4) that will be inserted in the intervertebral space (5) defined between two neighbouring vertebrae (2, 3) to be mutually secured, in order to restore the height and the angle of the lordosis of the vertebral segment defined by the two neighbouring vertebrae;
a stabilising plate (17) equipped with at least one passage hole (18) for an anchoring screw (19) at each of its ends, the plate (17) and the implant (4) being provided with mutual assembly means (21) such that after assembly, the stabilising plate (17) extends on each side of the implant to enable the stabilising plate to be anchored on the neighbouring vertebrae through the screws; and spacing means (30) interposed between the stabilising plate (17) and the implant (4), to enable the stabilising plate to be positioned at a specific distance relative to the implant,
the system being **characterised in that** the spacing means are composed of at least one spacer bushing (31) and **in that** the implant presents a reception part (33, 56) for receiving a complementary part (34, 55) presented by the spacer bushing (31) that is stopped between the stabilising plate (17) and the implant (4) so as to determine by its length, the distance between the stabilising plate (17) and the implant (4).

2. A system according to claim 1, **characterised in that** the reception part (33) is made by an entry cone.

3. A system according to claim 1 or claim 2, **characterised in that** the spacer bushing (31) is a nut having a prismatic shape and cooperating with a part (33) for reception of the implant (4).

4. A system according to one of claims 1 to 3, **characterised in that** the assembly means (21) are provided with support means for the plate allowing angular orientation of the plate (17) relative to the implant (4).

5. A system according to claim 1, **characterised in that** the spacer bushing (31) is fixed to the stabilising plate (17).

6. A system according to claim 1, **characterised in that** the spacer bushing (31) comprises assembly means on the implant, allowing adjustment of the distance between the stabilising plate (17) and the implant (4).

7. A system according to claim 6, **characterised in that** the spacer bushing (31) is fixed to the implant (4) and comprises notches (55) that cooperate with complementary notches (56) formed on the implant (4).

8. A system according to claim 5, **characterised in that** the spacer bushing (31) is put into place on an assembly screw (22) forming part of the assembly means (21) and designed to pass through the plate through a passage hole (24) bordered by a stop shoulder (25) arranged to hold the head (221) of the assembly screw (22) and to cooperate with at least one threaded hole (23) arranged in the implant.

9. A system according to claim 8, **characterised in that** the threaded hole (23) is formed on the anterior wall (6) of the implant, this wall having a convex curvature in the transverse plane and extending by connecting walls (8, 9) with a convex curvature, to a posterior wall (7) with a concave curvature to leave the vertebral canal free.

10. A system according to claim 9, **characterised in that** each connecting wall (8, 9) is arranged to contain a threaded hole (23) surrounded, like the threaded hole (23) formed in the anterior wall (6), by an assembly groove (40) designed to cooperate with two assembly dog pins (49) provided on the head (45) of a gripping instrument (44) of the implant (4), also comprising a threaded rod (47) designed to cooperate with a threaded hole (23) in the implant.

11. A system according to claim 9, **characterised in that** the posterior wall (7) is fitted with a radio-opaque element (35) opening up on the external face of the said wall, adapted so that the position of the implant within the intervertebral space (5) along the sagittal plane (S) can be detected.

12. A system according to claim 1, **characterised in that** the stabilising plate (17) is provided with anti-expulsion means (20) for the anchoring screws (19), installed on the plate (17) through moving guide means and adapted to occupy a first position in which the passage cross-section of the holes (18) for reception of the anchoring screws (19) is left free, and a second position, in which the holes (18) are partially closed off, in order to form a stop for the heads of screws.

## Patentansprüche

1. Stabilisiertes System zur intersomatischen Fusion für Wirbel, von der Art umfassend:
ein intersomatisches Implantat (4), das dazu bestimmt ist, in den Intervertebralraum (5), der zwischen zwei benachbarten, fest miteinander zu verbindenden Wirbeln (2, 3) definiert ist, eingefügt zu werden, um die Höhe sowie den durch die beiden benachbarten Wirbel definierten Lordosewinkel des Wirbelsegments wiederherzustellen,
eine Stabilisierungsplatte (17), die an jedem ihrer Enden mit wenigstens einem Durchgangsloch (18) für eine Verankerungsschraube (19) versehen ist, wobei die Platte (17) und das Implantat (4) mit Mitteln (21) zum Verbinden untereinander versehen sind, so daß nach dem Verbinden die Stabilisierungsplatte (17) sich auf beiden Seiten des Implantats erstreckt, um das Verankern der Stabilisierungsplatte an den benachbarten Wirbeln mittels der Schrauben zu ermöglichen, sowie
Abstandsmittel (30), die zwischen der Stabilisierungsplatte (17) und dem Implantat (4) eingefügt sind, um zu ermöglichen, die Stabilisierungsplatte in einem bestimmten Abstand von dem Implantat zu positionieren,
**dadurch gekennzeichnet, daß** die Abstandsmittel von wenigstens einer Distanzhülse (31) gebildet sind und daß das Implantat einen Aufnahmeteil (33, 56) für einen ergänzenden Teil (34, 55) der Distanzhülse (31) aufweist, die zwischen der Stabilisierungsplatte (17) und dem Implantat (4) in Anschlag gebracht ist, um durch ihre Länge den Abstand zwischen der Stabilisierungsplatte (17) und dem Implantat (4) zu definieren.

2. System nach Anspruch 1, **dadurch gekennzeichnet, daß** der Aufnahmeteil (33) von einem Eintrittskegel gebildet ist.

3. System nach Anspruch 1 oder 2, **dadurch gekennzeichnet, daß** die Distanzhülse (31) eine Mutter ist, die eine Prismenform (34) aufweist, welche mit dem Aufnahmeteil (33) des Implantats (4) zusammenwirkt.

4. System nach einem der Ansprüche 1 bis 3, **dadurch gekennzeichnet, daß** die Verbindungsmittel (21) mit Mitteln zum Tragen der Platte versehen sind, die eine winkelmäßige Ausrichtung der Platte (17) gegenüber dem Implantat (4) zulassen.

5. System nach Anspruch 1, **dadurch gekennzeichnet, daß** die Distanzhülse (31) an der Stabilisierungsplatte (17) befestigt ist.

6. System nach Anspruch 1, **dadurch gekennzeichnet, daß** die Distanzhülse (31) Mittel für das Anbringen an dem Implantat umfaßt, die ein Einstellen des Abstandes zwischen der Stabilisierungsplatte (17) und dem Implantat (4) gewährleisten.

7. System nach Anspruch 6, **dadurch gekennzeichnet, daß** die Distanzhülse (31) an dem Implantat (4) befestigt ist und Rastkerben (55) aufweist, die mit einer an dem Implantat (4) ausgebildeten ergänzenden Rastenanordnung (56) zusammenwirken.

8. System nach Anspruch 5, **dadurch gekennzeichnet, daß** die Distanzhülse (31) auf eine Verbindungsschraube (22) aufgesteckt ist, die zu den Verbindungsmitteln (21) gehört und dazu bestimmt ist, die Platte durch ein Durchgangsloch (24), das von einer Anschlagschulter (25) für den Kopf (221) der Verbindungsschraube (22) eingefaßt ist, zu durchgreifen und mit wenigstens einer in dem Implantat ausgebildeten Gewindebohrung (23) zusammenzuwirken.

9. System nach Anspruch 8, **dadurch gekennzeichnet, daß** die Gewindebohrung (23) in der vorderen Wand (6) des Implantats ausgebildet ist, die in der Querebene eine konvexe Krümmung aufweist und durch konvex gekrümmte Wände (8, 9) zum Verbinden mit einer hinteren Wand (7) fortgesetzt ist, welche eine konkave Krümmung aufweist, um den Wirbelkanal frei zu lassen.

10. System nach Anspruch 9, **dadurch gekennzeichnet, daß** jede Verbindungswand (8, 9) ausgebildet ist, um eine Gewindebohrung (23) zu umfassen, die - wie die in der vorderen Wand (6) ausgebildete Gewindebohrung (23) - von einer Verbindungsnut (40) gesäumt ist, die dazu bestimmt ist, mit zwei Verbindungszapfen (49) zusammenzuwirken, die an dem Kopf (45) eines Instruments zum Greifen (44) des Implantats (4) angeordnet sind, das auch einen Gewindestift (47) umfaßt, der dazu bestimmt ist, mit einer Gewindebohrung (23) des Implantats zusammenzuwirken.

11. System nach Anspruch 9, **dadurch gekennzeichnet, daß** die hintere Wand (7) mit einem röntgenstrahlenundurchlässigen Element (35) versehen ist, das an der Außenseite der Wand ausmündet, um zu ermöglichen, die Position des Implantats in dem Intervertebralraum (5) entlang der Sagittalebene (S) zu erfassen.

12. System nach Anspruch 1, **dadurch gekennzeichnet, daß** die Stabilisierungsplatte (17) mit Mitteln zur Ausstoßsicherung (20) der Verankerungsschrauben (19) ausgestattet ist, die mit Hilfe von Bewegungsführungsmitteln an der Platte (17) angebracht und geeignet sind, in einer ersten Position den Durchlaßquerschnitt der Löcher (18) zur Aufnahme der Verankerungsschrauben (19) frei zu lassen und in einer zweiten Position die Löcher (18) wenigstens teilweise zu verschließen, um einen Anschlag für die Köpfe der Schrauben zu bilden.
